# EUROPEAN PATENT SPECIFICATION

(11) **EP 2 399 522 B1**
(45) Date of publication and mention of the grant of the patent: **12.02.2020**
(21) Application number: 11169885.8
(22) Date of filing: 14.06.2011
(51) Int. Cl.: A61B 8/00

(54) **Monitoring system**
Überwachungssystem
Système de surveillance

(30) Priority: 28.06.2010 IT GE20100069
(43) Date of publication of application: 28.12.2011
(73) Proprietor: Esaote S.p.A., 16152 Genova (IT)
(72) Inventor: Cerofolini, Marino, I-52010 Subbiano (AR) (IT)
(74) Representative: Karaghiosoff, Giorgio Alessandro

(56) References cited:
- WO-A1-2006/097910
- WO-A1-2007/057826
- WO-A2-2007/131163

## Description

The present invention relates to a portable ultrasound monitoring system comprising a portable ultrasound imaging system which is in turn provided with an array of electroacoustic transducers, each one of which emitting ultrasound waves when powered with an electric excitation signal and generating an electric reception signal when impinged by an ultrasound wave or pulse generated for example by the reflection of ultrasound waves emitted therefrom. There are also provided means for generating said electric excitation signals, as well as means for receiving said electric reception signals and processing and storage units for said electric excitation and reception signals for setting the characteristics of emission pulses and for generating images and other parameters from reception signals. In addition communication means for the transmission and reception of the electric excitation and reception signals between said portable ultrasound system and at least a remote unit for displaying, storing and further processing as well as power supply means for the entire portable ultrasound system are provided.

Currently several types of portable ultrasound systems are known. Such systems have different shape characteristics corresponding to different portability and handiness levels similarly following the different types of portable computers and that is there are portable systems having a size similar to the so called notebook computers up to portable systems having a size similar to the so called tablet computers and up to systems of the type called Hand-held and suggesting such a size and handiness similar to those of the so called pocket-PC.

However all these systems are always designed for being extemporaneously used during an examination session and when the system is controlled and managed by a third person performing the examination on a patient.

In particular in the cardiology field, and in other fields as well, the patient needs to be subjected to a continuous examination for a certain period of time and during the normal daily life, in order to find pathologies having their effects under specific physical and/or psychological stress conditions. The so called Holter devices are currently known and these are devices that are carried for continuosly performing electrocardiograms. Sensors are applied to the patient which send signals to a continuous recording unit composed of a portable tape.

The developments concerning the electronic field and above all the miniaturization of electronic components and the reduction of their cost, together with the passage from analog to digital mode have allowed recording units to be made more and more small, lightweight and powerful, as well as power consumptions thereof to be reduced prolonging their life. Moreover functions for processing data detected by sensors have been added within said units.

The use of ultrasound imaging diagnostics has been considerably developed also in the cardiology field. The cardiographic imaging allows functional investigations to be made whose information are integrated with those obtained from the ECG leading to better and more accurate diagnostic overviews.

Document WO2006/097910 discloses a portable ultrasound monitoring system comprising an array of ultrasonic transducers and means for temporarily fastening wherein said means for temporarily fastening comprises pockets to arrange the elements of the ultrasound system.

Therefore, by means of relatively simple and inexpensive arrangements, there is the need of providing a monitoring system, in particular for diagnostic purposes, which is very easy to handle and which is able to fit different anatomical parts to be examined, and that, at the same time, is portable such to provide freedom of movement for the patient and which is able to easily interface both with other devices and with remote units for displaying and further processing detected data such that the fact that it is possible to perform examinations continuous in time and without the operator can be extended also in the ultrasound field, allowing the patient to live his/her normal daily life with an inconvenience as little as possible due to the provision of devices necessary for performing the examination.

The invention achieves the above aims by providing a monitoring system as defined in claim 1. Particular embodiments thereof are defined in the depenent claims.

The characteristics and advantages of the present invention will be more clear from the following description of some embodiments shown in annexed drawings wherein:
fig.1 shows a schematic possible embodiment of the monitoring system of the present invention;
fig.2 shows the means for temporary fastening to the body which belong to the monitoring system of the present invention according to a possible variant embodiment;
figs. 3a and 3b show two possible configurations of the fastening means of the monitoring system of the present invention;
figs. 4a and 4b show a possible variant embodiment of the monitoring system of the present invention;
figs. 5a and 5b are a perspective view and a side elevation view respectively of a possible configuration of the portable ultrasound system of the monitoring system of the present invention;
fig.6 schematically shows the monitoring system of the present invention where the portable ultrasound system is provided in combination with an electrocardiograph.
Figs. 7 and 8 like figures 4 and 5 show a variant embodiment of the ultrasound probe according to figures 4 and 5, which variant provides the probe to be divided into two parts that can be removably attached to each other.

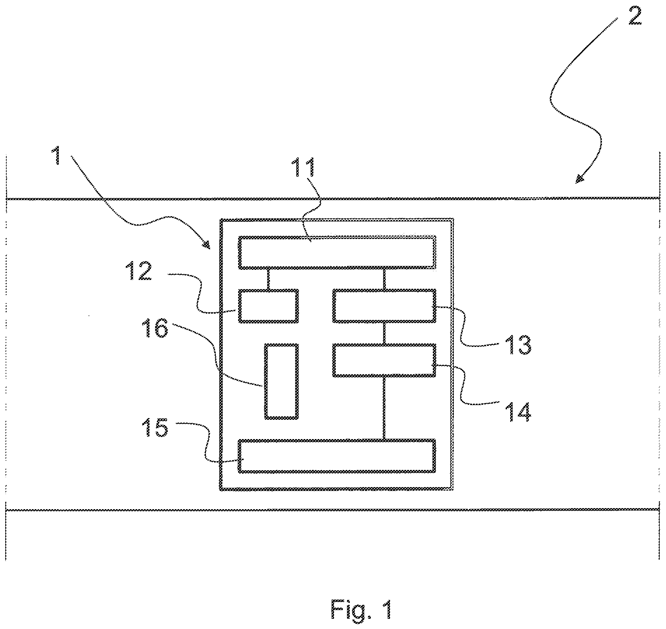

Figure 1 schematically shows a possible configuration of the monitoring system according to the present invention comprising a portable ultrasound system 1 which is in turn provided with an array of electroacoustic transducers 11, each one of which emitting ultrasound waves when powered with an electric excitation signal and generating an electric reception signal when impinged by an ultrasound wave or pulse generated for example by the reflection of ultrasound waves emitted therefrom. There are also provided means for generating electric excitation signals 12, as well as means for receiving electric reception signals 13 and processing and storage units 14 for said electric excitation and reception signals.

Such processing means can comprise means for generating image data from reception signals and/or also means for generating images from image data, which images can be displayed on local or remote displays. Processing means can also comprise units for processing/generating excitation pulses for transducers in order to control them in emitting ultrasound pulses having specific characteristics such as for example the duration, the frequency spectrum, the waveform, relative phase displacements among different transducer elements and other characteristics that can be typically modified in ultrasound pulses emitted from ultrasound probes.

Moreover there are provided communication means 15 for the transmission and reception of the electric excitation and reception signals between the portable ultrasound system 1 and at least a remote unit for displaying, storing and further processing, as well as power supply means 16 for the whole portable ultrasound system 1.

In order to place the portable ultrasound system 1 at specific locations of the body to be examined it is provided in combination with supporting means composed of further means 2 for temporary fastening to the body such that at least the array of transducers 11 is fastened in the specific position for performing the examination and therefore it is integral with the body, or the part thereof, to be examined.

According to possible variant embodiments of the monitoring system according to the present invention, the means 2 for temporary fastening to the body can be provided not only for fastening the array of transducers 11 in a specific position, but for allowing one or more of the costituent elements of the portable ultrasound system 1 to be additionally positioned.

In particular according to the variant embodiment of figure 1 all the constituent elements are arranged throughout the surface of the fastening means 2 and each element communicates with another one by communication lines 17.

Thus means for generating electric excitation signals 12, means for receiving electric reception signals 13, the processing and/or storage unit 14 for the electric excitation and/or reception signals, communication means 15 and power supply means 16 as well as the array of transducers 11 are fastened by the fastening means 2.

Communication lines 17 are provided integrated within the fastening means 2 too and transmit data among the several elements of the ultrasound system 1 by using cables.

According to a variant embodiment the communication lines allow the several constituent elements to communicate in wireless mode, for example by using radio wave transmission.

It is also possible to provide an interface in combination with communications means 15 such that data detected by the portable ultrasound system 1 can be transmitted to a remote display unit. Like communication lines 17, the transmission can be performed by using cables or according to a wireless mode, as a consequence such interface can be composed of a connector connected to a predetermined input of the remote unit, or can be composed of a radio wave antenna for wireless transmission. A further variant embodiment provides such interface to be composed of a storage device, which, in the perspective of reducing the overall dimensions, can be composed of a memory card of the Secure Digital type or the like, which records data detected by the monitoring system 1, stores them and displays them once it is inserted within the remote display unit.

In figure 1 the fastening means are schematically shown as a band to be wound and fastened around the body under examination with sensors and particularly with electroacoustic emitting and receiving transducers arranged at the organ to be examined that is at the anatomical window for performing the ultrasound scan of the organ to be examined. As it will be described below the band will have closure and tensioning means for example in the form of buckles and/or devices for removably interlocking the two ends of the band which can comprise also devices known under the brand name of Velcro® .

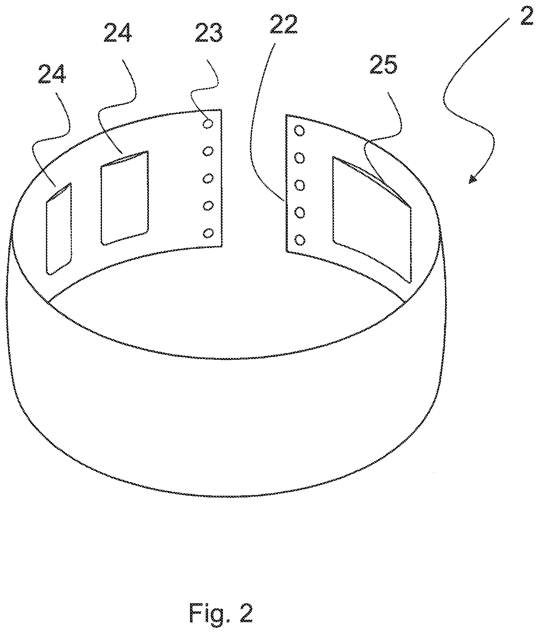

Figure 2 shows a particular configuration of the fastening means 2, wherein such means are composed of garment-like wearable elements. With a particular reference to figure 2, the fastening means 2 are composed of a band element intended for surrounding at least partially the part of the body to be monitored. Such element is open and has two ends 21 and 22 engaging one another by being at least partially overlapped along a closure composed of snap fasteners 23.

Possible embodiments of the closure for such element can be standard closures used in the clothing industry, such as for example any buttons, zip fasteners or hook-and-loop elements of the Velcro® type.

Moreover the fastening means 2 of figure 2 on the inside thereof, at the part in contact with the body to be examined, have one or more housing pockets 24 intended for housing and keeping in place one or more of the costituent elements of the portable ultrasound system. Inside the fastening means 2 there are also provided elements 25 intended for keeping one or more of the costituent elements of the portable ultrasound system in a fixed position too: such elements are arranged throughout the inner surface, or a portion thereof, of the fastening means 2 and are composed of a first part that is integral with the band and cooperating with and engaging a second part, which is attached to the several elements of the portable ultrasound system. In particular in figure 2 such elements 25 are composed of hook-and-loop elements of velcro type, but a possible variant embodiment for example provides the use of the two parts of snap fasteners.

Likewise a variant embodiment of the fastening means 2 according to the present invention, provides the housing pockets 24 not to be fixed, but to be composed of pockets cooperating with the inner part of the fastening means 2 by a coupling interface that can be composed of removable temporary fastening means, such as for example the two parts of a snap fastener or two cooperating and hook-and-loop detachable fastening elements of the type known under the brand name of Velcro® .

Therefore it is possible to provide all the inner surface of the temporary fastening means 2 to be composed of Velcro type material, such that the housing pockets 24 can be adjusted along the surface such to fit different patient morphologies and different body parts to be examined.

As an alternative one or more strip-like regions can be provided wherein one of the removable fastening elements of the means for removably fastening the pocket or pockets throughout the garment is provided, while the other element is provided on the removable pockets.

The above characteristics allow the positioning especially of sensors and particularly of the arrays of emitting and receiving transducers to be optimized with respect to the body of the patient a wearable garment fitting not only the patient size, but also morphological and anatomical differences that usually occur from one patient to another patient and therefore signals obtained by the examination are optimized.

Obviously it is also possible to provide the garment to have a plurality of pockets firmly attached to the garment each one at a different location.

A further embodiment provides such housing pockets 24 on the surface in contact with the fastening means 2 to have through loops slidably engaging along a belt that is provided attached to the surface of the fastening means 2, such that such housing pockets 24 can be adjusted throughout the surface.

The band element forming the fastening means 2 is preferably composed of elastic material and likewise according to a further improvement it can be a closed, endless continuous element fitting the size of the different bodies, or part thereof, to be examined by means of the elastic properties of the used material allowing the several elements of the ultrasound system 1 to be integral with the part of the body to be examined and not to be subjected to changes in their positions due to movement during the normal walking motion, while being highly capable of fitting any part of the body to be examined.

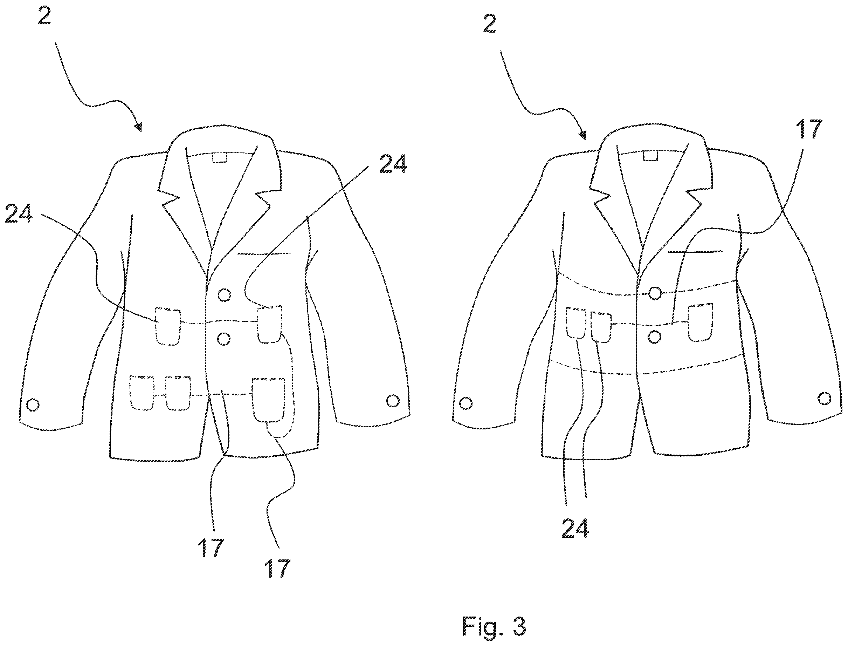

Figures 3a and 3b show two particular configurations of the monitoring system according to the present invention where the fastening means 2 are composed of garment-like elements. Particularly in figures 3a and 3b the garment is a jacket, but further embodiments can provide the fastening means 2 to be waistcoat, shirt, T-shirt, bra or any garment.

In figure 3a the costituent elements of the portable ultrasound system described in figure 1, are arranged throughout the inner surface of the whole garment, communicating each other by means of communication lines 17 and are kept in a fixed position by means of housing pockets 24.

As described above such housing pockets 24 can be provided in a fixed position or can be movable throughout all or a part of the inner surface of the jacket. In this last case such housing pockets 24 are composed of pockets cooperating with the inner part of the garment by means of a coupling interface that can be composed of detachable elements, such as for example the two parts of a snap fastener or two hook-and-loop elements of Velcro type.

As an alternative such housing pockets 24 on the surface in contact with the inner surface of the jacket have through loops slidably engaging along a belt that is provided attached to the inside of the jacket, such that such housing pockets 24 can be adjusted throughout the surface.

Figure 3b instead shows a jacket partially composed of a band forming the fastening elements 2 and having all the characteristics previously described with reference to figure 2.

It is also possible to provide an interface connected to one or more of the constituent elements of the ultrasound system 1, such that data detected thereby can be transmitted to a remote display unit. Like communication lines 17, the transmission can be performed by using cables or according to a wireless mode, as a consequence such interface can be composed of a connector connected to a specific input of the remote unit, or can be composed of a radio wave antenna for wireless transmission.

A further variant embodiment provides such interface to be composed of a storage device, which, in the perspective of reducing the overall dimensions, can be composed of a memory card of the Secure Digital type or the like, which records data detected by the monitoring system 1, stores them and displays them once it is inserted within the remote display unit.

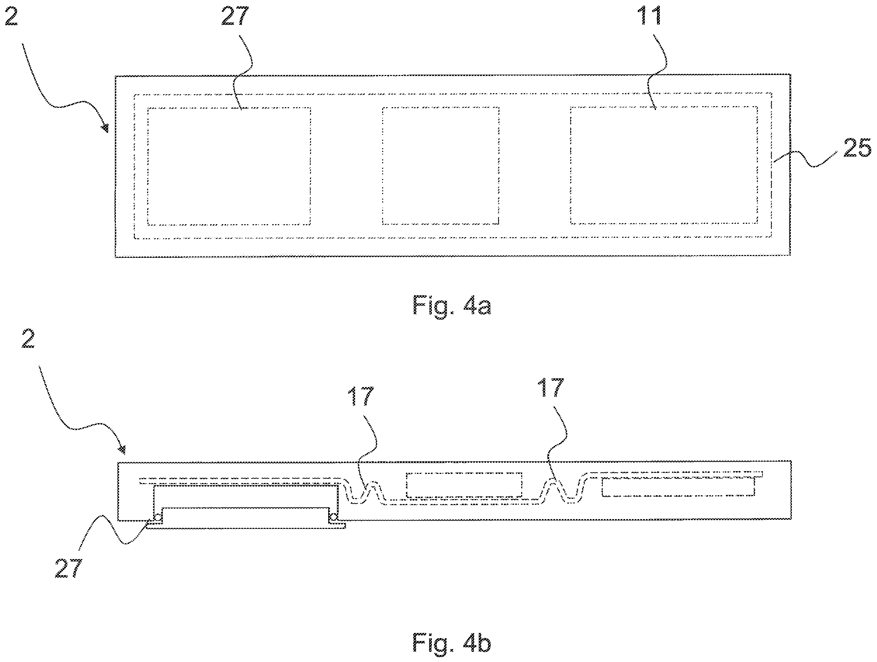

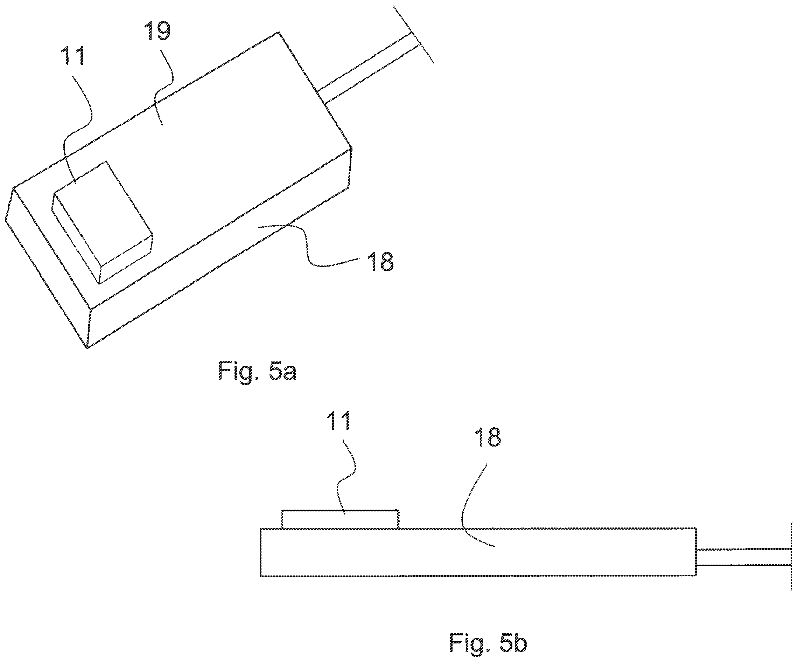

Figures 4 and 5 show a particular configuration of the portable ultrasound system 1 of the monitoring system of the present invention where the ultrasound system is composed of an ultrasound probe and of control electronics.

The ultrasound probe has an outer case having two side thinner and longer faces 18, and two wider and longer faces 19, parallel each other and oriented with their length dimension in the direction of a longitudinal axis. Moreover the array of transducers 11 is provided fitted on one of the two wider faces 19 such that the two faces 19 are oriented substantially perpendicularly to the direction of propagation of the acoustic beam emitted from the probe.

According to a first embodiment, both the ultrasound probe and the control electronics are housed within the housing pockets of the fastening means and there are several possible configurations providing the constituent elements of the portable ultrasound system 1 inside the ultrasound probe and/or the control electronics, and these are all the subject of the present invention.

In particular only the array of transducers 11 can be provided within the probe, that by means of communication lines 17 receives and sends signals from and to the control electronics and consequently means for generating electric excitation signals 12, means for receiving electric reception signals 13, the processing and/or storage unit 14 for the electric excitation and/or reception signals, communication means 15, and power supply means 16 are included therein.

As an alternative it is possible to provide the probe to include both the array of transducers 11 and the processing and/or storage unit 14 for the electric signals and to keep inside the electronic control unit means for generating the electric excitation signals 12, means for receiving the electric reception signals 13, communication means 15 and power supply means 16. Otherwise the array of transducers 11, the processing and/or storage unit 14, signal generating means 12 can be provided inside the probe and the function of the control electronics can be only to power the whole system and to communicate the data processed by the probe to a remote unit, or as previously described, to save such data on a storage unit.

In particular the power supply means 16 can be composed of a source generating and storing electrical power, of the battery type or the like, that can be rechargeable, with the recharging circuits integrated within the means for temporary fastening to the body too.

As a further embodiment it is possible to provide the probe to directly communicate with a remote display unit without the aid of the electronic control unit: in this case the probe will include the array of transducers 11, means for generating electric signals 12, means for receiving the electric reception signals 13, the processing and/or storage unit 14, power supply means 16 and communication means 15, that will be connected to an interface allowing data detected by the probe to be transmitted to a remote display unit according to the methods described above.

As an alternative it is possible to provide communication means 15 to transmit data to the remote display unit according to a wireless mode: apart from the case in which both communication means 15 and the processing unit 14 are inserted within the probe or the control electronic unit, the fact of providing inside the processing unit a unit intended for reducing the bandwidth occupied by the transmitted signal is preferred such that the transmission between the portable ultrasound system 1 and the remote display unit is facilitated. An example may be a unit generating video signals obtained from images deriving from the processing of received signals.

An example of the technology concerning a wireless probe that has to be considered as not limitating the several possible configurations of the system is described in EP 2164398 and WO2008/066681.

Figure 5 schematically shows a possible variant embodiment of the monitoring system of the present invention where the portable ultrasound system 1 is provided in combination with an electrocardiograph 3.

The monitoring device 1 is composed of an ultrasound probe 3 and control electronics 31, according to characteristics widely described above in figure 5, and it is provided in combination with an electrocardiograph device.

Such electrocardiograph device is composed of a signal recording unit 4 recording signals transmitted from one or more electrodes 41.

With a particular reference to figures 5a and 5b, both the portable ultrasound system and the electrocardiograph device are supported by means 2 for temporary fastening to the body, which have all the characteristics concerning the fastening means previously described in figure 2.

In particular the ultrasound probe 3, the electronic control unit 31 and the recording unit 4 are housed within the housing pockets 24, while sensors 41 are supported by elements 25 which are composed of hook-and-loop elements of Velcro type cooperating one with the other a first part of which being integrally attached to each electrode 41, while a second part is provided on the inner surface of the fastening means 2.

Even in this case both the housing pockets 24 and the elements 25 have all the characteristics described in figure 2.

According to a further embodiment the electrodes 41 are fitted on the recording unit 4, which is consequently integral therewith such that both the recording unit 4 and the electrodes 41 are placed within the same housing pocket 24 or are fitted on the same element 25.

Finally according to an improvement of the present invention it is possible to provide the recording unit 4 of the electrocardiograph to be provided within the electronic control unit 31 of the ultrasound probe 3. In this case it is possible to provide processor means inside the electronic control unit for executing a logic processing program intended for setting a connection among data received from the ultrasound probe 3 and from the electrocardiograph.

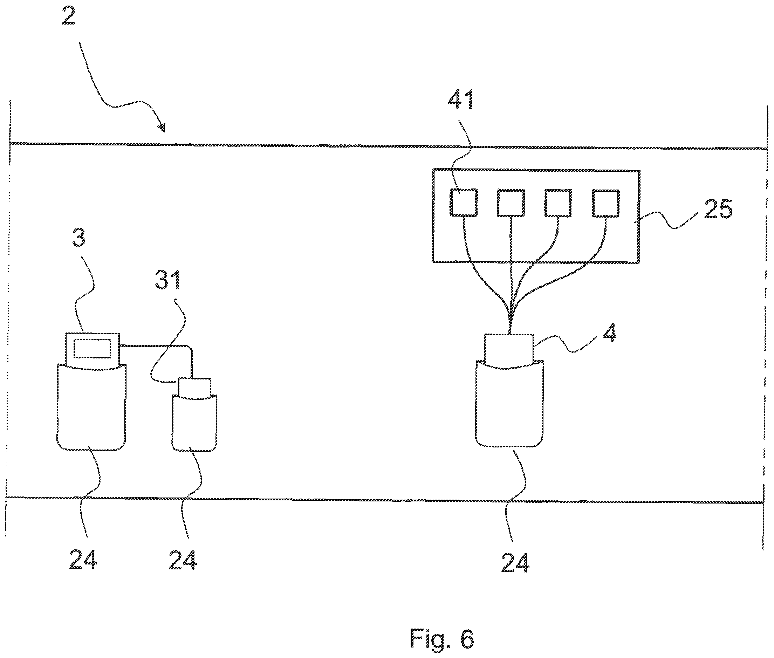

Even in this case the monitoring system according to the present invention that, with a particular reference to figure 6, provides the use of two different monitoring devices, the ultrasound probe and of the electrocardiograph, can use an interface for transmitting data detected by the two different monitoring devices to a remote display unit. The transmission can occur by using cables or in wireless mode, consequently such interface can be composed of a connector connected to a specific input of the remote unit, or can be composed of a radio wave antenna for the wireless transmission. A further variant embodiment provides such interface to be composed of a storage device, which, in the perspective of reducing the overall dimensions, can be composed of a memory card of the Secure Digital type or the like, which records the received data, stores them and displays them once it is inserted within the remote display unit.

On the remote display unit will be possible to display in a diversified manner the received signals, or it will be possible to set a functional connection among the received signals by means of the processing program provided inside the control unit in common to the two monitoring devices.

Finally, the two monitoring devices can also advantageously display the received and recorded signals independently from the remote display unit, by means of a screen fitted on the control electronics 31 and on the recording unit 4 respectively.

With reference to the several shown embodiments it is possible to provide the several operating units of the diagnostic systems, such as the ultrasound system, the ECG system or the ultrasound and ECG combined system to be divided in several separated physical units and to be arranged at different locations with reference to the extension and the shape of the fastening means, a dedicated housing pocket being provided for each physical unit having dimensional and electrical properties adapted for the needs of the units they have to house. Obviously the cables connecting said physical units have to be integrated within or associated to the fastening means.

To this aim said cables can be provided housed into suitable channels called coulisse provided within the fastening means and within which the cables can be threaded and unthreaded or firmly inserted. The latter at their ends in turn will have terminals connecting to corresponding complementary connectors provided on the physical units.

A particular embodiment can provide a plurality of parallel coulisses housing the connection cables along the extension of the fastening means and for example parallely to the extension of the removably fastening means of the pockets 24, which coulisses have a wall opposite to the means 2 for fastening to the patient body which is not continuous, that is it is provided with a plurality of inlet and outlet apertures for the cable housed within the coulisse. Said row of apertures allows the cable to enter and come out from the coulisse at different locations thereof and therefore the length of the cable housed within the coulisse to be adjusted to the variable distances among the physical units since said units can be placed at a plurality of different positions with reference to the shape and the surface extension of the means for fastening the system to the body under exmination.

When pockets are fixed, then the coulisses can be such that the pockets are directly connected one another.

As an alternative, it is possible to provide cables whose outer covering jacket has an outer surface covered or provided at least at partial areas with removable fastening means of the Velcro® type or the like which are intended for cooperating with the complementary removable interlocking means provided on the wearable fastening means.

Likewise a possible variant embodiment of the monitoring system of the present invention provides the portable ultrasound system to be provided in combination with elements for temporary fastening to the body composed of a band element comprising rigid elements in combination with flexible elements.

Rigid elements can act as the housing pockets 24 and can allow one or more of the elements of the portable ultrasound system to be housed, while coulisse-like seats are housed within the flexible elements for receiving the communication lines 17 for the communication among the several constituents.

Likewise the several described embodiments, both the portable ultrasound system and the means for temporary fastening to the body have all the characteristics already previously widely described, in particular whether the ultrasound system is a wireless ultrasound probe, it is advantageous for the rigid elements to have slots therein. Such slots allow the battery to be inserted within the probe without the need of taking the probe out from the housing, or they can be apertures for connecting the whole system to interfaces intended for example for the connection to an external mains supply for recharging the battery, or for further devices storing the data detected by the probe.

Moreover flexible elements can not only house the communication lines, but they can be the communication contacts among the several components of the portable ultrasound system: such configuration allows a modular structure of the fastening elements to be obtained comprising a series of rigid elements housing the components of the portable ultrasound system which are alternated with the flexible elements.

Finally preferably, but not exclusively, the band element, particularly the rigid elements, is composed of an elastically deformable material, for example rubber materials, allowing the components housed therein to be protected while remaining highly capable of fitting the body part and/or the patient to be examined.

In particular figures 4a and 4b show a possible variant embodiment of the monitoring system of the present invention. According to such configuration the several components of the portable ultrasound system are provided in combination with means 2 for temporary fastening to the body composed of a band element: such components are fitted embedded within the material of the band element 2.

Thus the band element 2, in addition to act for the temporary fastening to the body acts also as a casing for components of the portable ultrasound system thus replacing the real casing containing them. The means for temporary fastening to the body in the inside part in contact with the body can have a slot allowing the array of transducers 11 to contact the body part to be examined. The array of transducers 11 is connected to the remaining components, already described above, of the ultrasound system which are integrated within the band element and connected each other by means of the communication lines 17 also integrated within the same element. Advantageously the band element provides a containing enclosure element 27 that can be opened for housing the battery, opening on the top in the direction opposite to the inner part in contact with the body under examination, for taking out and introducing the battery and/or for connecting the interfaces already described above.

According to an improvement of the variant embodiment shown in figures 4a and 4b the material of the band element 2 is a deformable and waterproof material, for example a rubber material, while the containing enclosure element 27, for housing the battery has a watertight closure such to insulate the whole system against possible infiltrations.

The slot is likewise provided on the inner surface of the fastening means in contact with the body and it surrounds the array of transducers 11 at the side perpendicular to the end side thereof, which is in contact with the body part to be examined too, such that the end side is flush with the rubber material of the fastening means 2 surrounding the array of transducers. Moreover the slot is made in a watertight manner such that the interface part between the rubber part and the array of transducers 11 does not allow liquids to penetrate into the means 2 for temporary fastening to the body.

With a particular reference to figures 4a and 4b, the array of ultrasound transducers 11 is also embedded inside the band element 2, without providing any slots such that the rubber element of the band element 2 can be passed by ultrasounds and it acts both as the protection and as the coupling means between the array of transducers 11 and the body part under examination.

According to an improvement shown in figures 4a and 4b, an inner receiving unit 28 is provided, embedded within the fastening means 2 too, for the communication between the portable ultrasound system and one or more of the remote control devices intended for example for setting operation parameters of the portable ultrasound system. As an alternative to said inner receiving unit 28, it is possible to provide a push-button panel made within the fastening means 2 composed of pressure elastic areas made as one piece with the rubber material of the band 2.

Similarly to what said above even the portable ultrasound system and the means for temporary fastening to the body described in figures 4a and 4b have all the characteristics of the several previously described embodiments of the monitoring system of the present invention.

Again according to a possible variant, with reference to the embodiment of figures 5a and 5b, it is possible to provide the ultrasound probe to be made of two parts one of which composed of the array of tranducers, fitted on a bearing element provided with a multipolar terminal for the connection to a complementary connection terminal provided on the remaining part of the probe which is intended for housing the electronic units the two connectors establishing a communication between the transducers of the array of transducers and said electronic units. By means of such arrangement it is possible to replace the array of transducers adapting it to the examination requirements. In particular therefore on the same probe using common electronic units it is possible to fit linear or two-dimensional arrays of transducers and having a different number of elements or dimensional extensions.

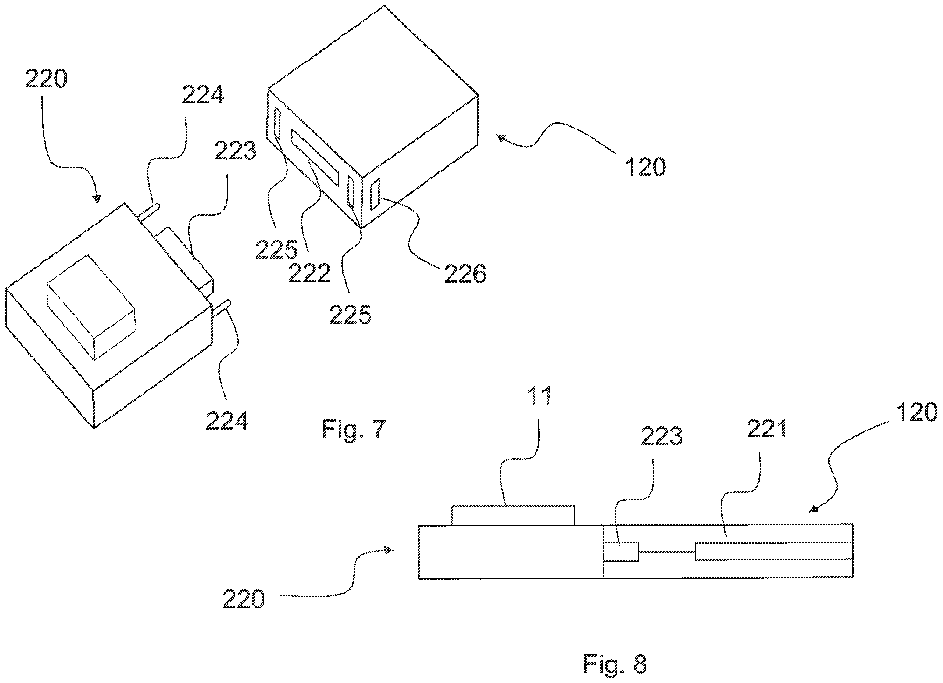

An example is shown in figures 7 and 8. Such example has not to be considered as a limitation. Several configurations are possible and fall within the range of the person skilled in the art as an obvious choice among different options.

In particular the array of transducers that is shown can be replaced without the need of replacing also a part of the probe case. However if the replacement is performed by people not skilled in the probe construction the shown solution will be more convenient and safe.

In the example of figures 7 and 8, a probe of the type described in figures 5a and 5b has a case 20 divided into two parts 120 and 220 that can be mechanically removably coupled together for example by means of removable interlocking means provided on the two parts 220 and 120 of the case 20. Said means can be of any type and can be selected among the several variants known to and available to the person skilled in the art.

In figures 7 and 8 said means are schematically shown by male elements 224 having an extension in the direction for mutually connecting/releasing the two case parts 120, 220 having a tooth end on one case part 220 intended for being engaged behind a mating element provided within an interlocking seat 225 in the other case part 120. Button means 226 can be controlled from the outside in order to bend the male elements 224 in a position releasing the tooth end thereof and so allowing them to be taken out from the seats 225 and allowing the two parts of the case 220 and 120 to be moved away one with respect to the other.

A case part 220 bears the array of transducers 11. Each transducer element is connected by a line with a pole of a connection terminal 223 (in this case a male) provided on the case part 220 at the face intended to adhere against the corresponding face of the case part 120 with the two case parts 120, 220 in a coupled condition.

The connection terminal 223 has an electrical pole for each communication line of each transducer element of the array 11 and it cooperates with a complementary connection terminal 222 provided on the face of the case part 120 intended to contact the face of the other case part 220.

The position of the two connection terminals 222, 223 and even the direction for being mutually engaged and released is advantageously the same necessary for mechanically coupling and releasing said two case parts 120, 220. By this arrangement by joining together the two case parts 120 and 220 the electrical contact between the two connection terminals 222, 223 and therefore between the individual transducer elements of the array of transducers 11 and the processing electronics 221 is automatically established. Such electronics can be configured according to one or more of the several variants described above or known from the prior art with reference to the greater or lower integration of operating units into the ultrasound probe.

As it is clear the construction allows the array of transducers to be replaced with other array of transducers that can be different as regards the number of transducers and the arrangement thereof inside the array. Considering the fact of making the electronics 221 and the connectors 222, 223 all equal each other and correspondingly to the array of transducers 11 providing as many allowable transducer elements as possible, the probe will be adapted in a very simple way to the provision of a different number of transducers in the array of transducers, by simply causing the contacts or poles of the connector 223 of the case part 220 bearing the array of transducers 11 to be not operating, therefore the channels of the electronic unit 221 corresponding to those of the non operating contacts of the connector 223 are not operating too.

## Claims

1. Portable ultrasound monitoring system comprising a portable ultrasound imaging system (1) provided with:
an array of electroacoustic transducers (11), each one of which emitting ultrasound waves when powered with an electric excitation signal and generating an electric reception signal when impinged by an ultrasound wave or pulse generated for example by the reflection of ultrasound waves emitted therefrom,
means for generating said electric excitation signals (12), as well as means for receiving said electric reception signals (13),
a processing and/or storage unit (14) for said electric excitation and/or reception signals,
communication means (15) for the transmission and reception of said electric excitation and/or reception signals between said portable ultrasound system (1) and at least a remote unit for displaying, storing and further processing,
power supply means (16) for said portable ultrasound system (1),
said portable ultrasound imaging system (1) being provided in combination with supporting means, composed of means (2) for temporarily fastening at said array of transducers (11) to the body in the predetermined position for performing the examination and of means for temporarily fastening the remaining elements of said portable ultrasound system (1) to the body,
communication lines (17) between said elements and said array of electroacoustic transducers (11) being provided,
said portable ultrasound imaging system (1) being composed of an ultrasound probe and of an electronic control unit,
said probe comprising at least said array of electroacoustic transducers (11)
said means (2) for temporary fastening to the body, on the inside thereof, in the portion in contact with the body under examination, have one or more housing pockets (24) intended for keeping said ultrasound probe and/or said electronic control unit in a fixed position,
each pocket being arranged at predetermined locations within said fastening means (2) such that both one or more elements of said portable ultrasound imaging system (1) and said communication lines (17) are fastened and/or integrated,
**characterized in that**
the said pockets and the inner side of the said means (2) for temporary fastening to the body being provided with a coupling interface composed of detachable elements selected amonge the following alternatives:
a two parts of a snap fastener;
a two hook-and-loop elements of Velcro type;
a belt on the inside of the means (2) for temporary fastening to the body in combination with through loops on the pockets engaging the said belt,
so that the pockets can be movable throughout all or part of the inner surface of the said means (2) for temporary fastening to the body.

2. Portable ultrasound monitoring system according to claim 1, wherein said ultrasound probe comprises an outer case having two side thinner and longer faces (18) and two wider and longer faces (19), parallel each other and oriented with their length dimension in the direction of a longitudinal axis,
said array of transducers (11) being fitted on one of said two wider faces (19) such that said two faces are oriented substantially perpendicularly to the direction of propagation of the acoustic beam emitted from the probe.

3. Portable ultrasound monitoring system according to one or more of the preceding claims, wherein in combination with said ultrasound probe a further monitoring device composed of an electrocardiograph is provided.
said electrocardiograph being composed of a recording unit (4) in communication with at least an electrode (41).

4. Portable ultrasound monitoring system according to one or more of the preceding claims, wherein said recording unit (4) is provided inside said electronic control unit (31) of the portable ultrasound imaging system (1).

5. Portable ultrasound monitoring system according to one or more of the preceding claims, wherein said means (2) for temporary fastening to the body are composed of garment-like wearable elements.

6. Portable ultrasound monitoring system according to one or more of the preceding claims, wherein said fastening means (2), on the inside thereof, in the portion in contact with the body under examination, have Velcro hook-and-loop elements intended for keeping said portable ultrasound imaging system (1) in a fixed position.

## Patentansprüche

1. Tragbares Ultraschallüberwachungssystem umfassend ein tragbares Ultraschallbildgebungssystem auf (1) mit einer Anordnung von elektroakustischen Wandlern (11), die jeweils Ultraschallwellen aussenden, wenn sie mit einem elektrischen Anregungssignal beaufschlagt werden, und ein elektrisches Empfangssignal erzeugen, wenn auf sie eine Ultraschallwelle oder ein Ultraschallimpuls einfällt, die/der beispielsweise durch Reflexion von aus diesen abgestrahlten Ultraschallwellen erzeugt werden, Mittel zum Erzeugen der elektrischen Anregungssignale (12) sowie Mittel zum Empfangen der elektrischen Empfangssignale (13), eine Verarbeitungs- und/oder Speichereinheit (14) für die elektrischen Anregungs- und/oder Empfangssignale, Kommunikationsmittel (15) zum Senden und Empfangen der elektrischen Anregungs- und/oder Empfangssignale zwischen dem tragbaren Ultraschallsystem (1) und wenigstens einer Ferneinheit zum Anzeigen, Speichern und Weiterverarbeiten, Energieversorgungsmittel (16) für das tragbare Ultraschallsystem (1), wobei das tragbare Ultraschallbildgebungssystem (1) in Kombination mit Stützmitteln vorgesehen ist, die aus Mitteln (2) zur vorübergehenden Befestigung der Anordnung von Wandlern (11) an dem Körper in der vorbestimmten Position zur Durchführung der Untersuchung und aus Mitteln zur vorübergehenden Befestigung der übrigen Elemente des tragbaren Ultraschallsystems (1) an dem Körper besteht, wobei Kommunikationsleitungen (17) zwischen den Elementen und der Anordnung elektroakustischer Wandler (11) vorgesehen sind, wobei das tragbare Ultraschallbildgebungssystem (1) aus einer Ultraschallsonde und einer elektronischen Steuereinheit zusammengesetzt ist, wobei die Sonde zumindest die Anordnung elektroakustischer Wandler (11) umfasst, wobei die Mittel (2) zur vorübergehenden Befestigung an dem Körper an dessen Innenseite in dem mit dem zu untersuchenden Körper in Kontakt stehenden Abschnitt eine oder mehrere Aufnahmetaschen (24) aufweisen, die dazu bestimmt sind, die Ultraschallsonde und/oder die elektronische Steuereinheit in einer festen Position zu halten, wobei jede Tasche an vorbestimmten Stellen innerhalb der Befestigungsmittel (2) derart angeordnet ist, dass ein oder mehrere Elemente des tragbaren Ultraschallbildgebungssystems (1) und auch die Kommunikationsleitungen (17) befestigt und/oder integriert sind, **dadurch gekennzeichnet, dass** die Taschen und die Innenseite der Mittel (2) zum vorübergehenden Befestigen an dem Körper mit einer Kopplungsschnittstelle versehen sind, die aus lösbaren Elementen zusammengesetzt ist, die aus den folgenden Alternativen ausgewählt sind:
zwei Teile eines Schnappverschlusses;
zwei Widerhaken- und Schlaufenelemente vom Typ Klettverschluss;
einen Gürtel an der Innenseite der Mittel (2) zum vorübergehenden Befestigen an dem Körper in Kombination mit Durchgangsschlaufen an den Taschen, die mit dem Gurt in Eingriff stehen, so dass die Taschen über die gesamte oder einen Teil der Innenfläche der Mittel (2) zur temporären Befestigung an dem Körper bewegbar sind.

2. Tragbares Ultraschallüberwachungssystem nach Anspruch 1, wobei die Ultraschallsonde ein äußeres Gehäuse mit zwei seitlichen dünneren und längeren Seiten (18) und zwei breiteren und längeren Seiten (19) umfasst, die parallel zueinander und mit ihrer Längenabmessung in Richtung einer Längsachse ausgerichtet sind, wobei die Anordnung von Wandlern (11) auf einer der zwei breiteren Seiten (19) derart angebracht ist, dass die zwei Seiten im Wesentlichen senkrecht zur Ausbreitungsrichtung des von der Sonde emittierten akustischen Strahls ausgerichtet sind.

3. Tragbares Ultraschallüberwachungssystem nach einem oder mehreren der vorhergehenden Ansprüche, wobei in Kombination mit der Ultraschallsonde eine weitere aus einem Elektrokardiographen bestehende Überwachungseinrichtung vorgesehen ist, wobei der Elektrokardiograph aus einer mit mindestens einer Elektrode (41) in Verbindung stehenden Aufnahmeeinheit (4) besteht.

4. Tragbares Ultraschallüberwachungssystem nach einem oder mehreren der vorhergehenden Ansprüche, wobei die Aufnahmeeinheit (4) innerhalb der elektronischen Steuereinheit (31) des tragbaren Ultraschallbildgebungssystems (1) vorgesehen ist.

5. Tragbares Ultraschallüberwachungssystem nach einem oder mehreren der vorhergehenden Ansprüche, wobei die Mittel (2) zur vorübergehenden Befestigung an dem Körper aus kleidungsartigen, tragbaren Elementen bestehen.

6. Tragbares Ultraschallüberwachungssystem nach einem oder mehreren der vorhergehenden Ansprüche, wobei die Befestigungsmittel (2) an ihrer Innenseite in dem mit dem zu untersuchenden Körper in Kontakt stehenden Abschnitt klettverschlussartige Widerhaken- und Schlaufenelemente aufweisen, die dazu bestimmt sind, das tragbare Ultraschallbildgebungssystem (1) in einer festen Position zu halten.

## Revendications

1. Système de monitorage à ultrasons portable comprenant un système d'imagerie à ultrasons portable (1) muni:
d'un réseau de transducteurs électroacoustiques (11), chacun émettant des ondes ultrasonores lorsqu'il est alimenté avec un signal d'excitation électrique et générant un signal de réception électrique lorsqu'il est atteint par une onde ultrasonore ou une impulsion générée par exemple par la réflexion des ondes ultrasonores émises par celui-ci,
de moyens pour générer lesdits signaux d'excitation électriques (12), ainsi que de moyens pour recevoir lesdits signaux de réceptions électriques (13),
d'une unité de traitement et/ou de stockage (14) pour lesdits signaux d'excitation et/ou de réception électriques,
de moyens de communication (15) pour l'émission et la réception desdits signaux d'excitation et/ou de réception électriques entre ledit système à ultrasons portable (1) et la au moins une unité éloignée pour l'affichage, le stockage et le traitement ultérieur,
de moyens d'alimentation (16) pour ledit système à ultrasons portable (1),
ledit système d'imagerie à ultrasons portable (1) étant prévu en combinaison avec des moyens de support, composés de moyens (2) de fixation temporaire au corps en correspondance dudit réseau de transducteurs (11) dans la position prédéterminée pour effectuer l'examen et de moyens de fixation temporaire des éléments restants dudit système à ultrasons portable (1) au corps,
étant prévues des lignes de communication (17) entre lesdits éléments et ledit réseau de transducteurs électroacoustiques (11),
ledit système d'imagerie à ultrasons portable (1) étant composé d'une sonde à ultrasons et d'une unité de commande électronique,
ladite sonde comprenant au moins ledit réseau de transducteurs électroacoustiques (11)
lesdits moyens (2) de fixation temporaire au corps, à l'intérieur de celui-ci, dans la partie en contact avec le corps examiné, présentent une ou plusieurs poches de logement (24) destinées à maintenir ladite sonde à ultrasons et/ou ladite unité de commande électronique en position fixe,
chaque poche étant agencée à des emplacements prédéterminés dans lesdits moyens de fixation (2) de sorte qu'un ou plusieurs des éléments dudit système d'imagerie à ultrasons portable (1) et lesdites lignes de communication (17) soient fixés et/ou intégrés,
**caractérisé en ce que**
lesdites poches et la face interne desdits moyens (2) de fixation temporaire au corps étant pourvues d'une interface de couplage composée d'éléments détachables sélectionnés parmi les alternatives suivantes:
un bouton à pression en deux parties;
deux éléments à crochets et boucles de type Velcro;
une ceinture à l'intérieur des moyens (2) de fixation temporaire au corps en combinaison avec les boucles traversantes sur les poches engageant ladite ceinture, de sorte que les poches puissent être déplacées le long de toute ou d'une partie de la surface intérieure des lesdits moyens (2) de fixation temporaire au corps.

2. Système de monitorage à ultrasons portable selon la revendication 1, dans lequel ladite sonde à ultrasons comprend un boitier extérieur ayant deux faces latérales plus fines et plus longues (18) et deux face plus larges et plus longues (19), parallèles les unes par rapport aux autres et orientées dans le sens de la longueur dans la direction d'un axe longitudinal,
ledit réseau de transducteurs (11) étant monté sur l'une desdites deux plus larges faces (19) de sorte à ce que les deux faces soient orientées sensiblement perpendiculairement à la direction de propagation du faisceau acoustique émis par la sonde.

3. Système de monitorage à ultrasons portable selon l'une ou plusieurs des revendications précédentes, dans lequel, en combinaison avec ladite sonde à ultrason, un autre dispositif de monitorage composé d'un électrocardiographe est fourni
ledit électrocardiographe étant composé d'une unité d'enregistrement (4) en communication avec au moins une électrode (41) .

4. Système de monitorage à ultrasons portable selon l'une ou plusieurs des revendications précédentes, dans lequel ladite unité d'enregistrement (4) est fournie à l'intérieur de ladite unité de commande électronique (31) du système d'imagerie à ultrasons portable (1) .

5. Système de monitorage à ultrasons portable selon l'une ou plusieurs des revendications précédentes, dans lequel lesdits moyens (2) de fixation temporaire au corps sont composés d'éléments portables ressemblant à des vêtements.

6. Système de monitorage à ultrasons portable selon l'une ou plusieurs des revendications précédentes, dans lequel lesdits moyens de fixation (2), à l'intérieur de celui-ci, dans la partie en contact avec le corps examiné, présentent des éléments à crochets et boucles de type Velcro destinés à maintenir ledit système d'imagerie par ultrasons portable (1) en position fixe.
